# EUROPEAN PATENT APPLICATION

(11) **EP 4 710 866 A1**
(43) Date of publication of application: **18.03.2026**
(21) Application number: 24200591.6
(22) Date of filing: 16.09.2024
(51) Int. Cl.: A61B 8/10, A61B 8/00

(54) **ULTRASOUND IMAGING SYSTEM AND METHOD**

(71) Applicant: ECHOPTIX GmbH, 80797 Munich (DE)
(72) Inventor: FARIDPOOYA, Koorosh, 3054VG Rotterdam (NL)
(74) Representative: Oancea, Andreas Christian

(57) **Abstract**

The invention concerns an ultrasound imaging system (10), comprising a patient interface device (12) having a contacting section (14) for contacting a subject, at least an ultrasonic probe (20), at least a robotic device (30) having a base portion (32) and a distal support portion (34) spaced from the base portion (32), and a control device (40), wherein the ultrasonic probe (20) is fixedly connected to the distal support portion (34), wherein the distal support portion (34) is movably coupled to the base portion (32) by means of one or more robotic arms (36), wherein the base portion (32) is connected to the patient interface device (12), and wherein the control device (40) is configured to control the robotic device (30) to move the ultrasonic probe (20) from a first position to a second position to at least partly scan the subject. Moreover, the invention concerns a method of imaging at least part of a subject by an ultrasound imaging system (10)

## Description

The present invention concerns an ultrasound imaging system, in particular, an ophthalmic ultrasound imaging system, as well as a method of imaging at least part of a subject, in particular, an eye of the subject by such an ultrasound imaging system.

In modern medicine, ultrasound imaging technology can be used for visualizing the interior of a patient's eye. More specifically, ultrasound imaging systems enable ophthalmologists to quickly and comparably accurately locate anatomical features as well as possible injuries such as hematoma inside the eye, which may cause severe vision loss. For this purpose, an examination method has been established in which the ophthalmologist requests the patient (subject) to close his/her eyes, after which the ophthalmologist brings an ultrasonic probe into contact with the eyelid of the patient. Then, at least one sectional image (a so-called B-scan image ("B-scan")) through the eye of the patient is generated. Subsequently, the ophthalmologist may ask the patient to look in a different direction. This line of vision change may allow for generating a further cross-sectional image without moving the ultrasonic probe. Thus, the ophthalmologist is provided with a solid information basis for making her/his diagnosis and determining a suitable treatment, if necessary.

Against this background, it is an object of the present invention to provide an ultrasound imaging system that enables even comparably inexperienced ophthalmologists to more efficiently and nevertheless accurately examine the patient's eye non-invasively. Furthermore, it is an object of the present invention to provide a corresponding method of imaging.

This object is solved by an ultrasound imaging system having the features of claim 1 as well as a method of imaging according to claim 16.

The ultrasound imaging system comprises a patient interface device having a contacting section for contacting a subject, at least an ultrasonic probe, at least a robotic device having a base portion and a distal support portion spaced from the base portion, as well as a control device. The ultrasonic probe is fixedly connected to the distal support portion. The distal support portion is movably coupled to the base portion by means of one or more robotic arms. The base portion is connected to the patient interface device. The control device is configured to control the robotic device to move the ultrasonic probe from a first position to a second position to at least partly scan the subject.

In particular, the ultrasound imaging system may be an ophthalmic ultrasound imaging system. Thus, the contacting section may be suitable for contacting the face of the subject (patient); the part of the subject scanned may be an eye. The scanning may be performed through on the open eye or through an eyelid. Further, the system may comprise a coupling medium for covering the (open or closed) eye. The coupling medium may comprise, for example, an ultrasonic gel.

The ultrasound imaging system according to the present invention thus enables examining the subject sonographically in an at least partially automated and thus comparatively fast, efficient and reproducible manner. In particular, the physician (e.g., ophthalmologist) may simply bring the patient interface device into a predetermined position in the proximity of the part of the subject to be scanned. Once the contacting section is accordingly in (direct or indirect) contact with the part of the subject (in particular, the face of the subject), the ultrasound imaging system may scan the subject under the control of the control device without the physician having to specifically manipulate the ultrasonic probe. Therefore, even little experienced physicians are supported with making precise and fast diagnoses, resulting in better medical care.

Moreover, the above advantages allow for reducing training times for physicians, resulting in high user acceptance. While the advantages are generally achieved regardless of the type of scan performed on the subject, that is, whether the scan is, e.g., an A-scan, a B-scan or a C-Scan, the more the ultrasonic probe is moved by the robotic device for performing such scan, the more prominent are the advantages of this technology.

In the context of the present specification, the term ultrasound imaging system (ultrasonic imaging system) refers to a system in which the images are reconstructed from ultrasonic waves detected by the ultrasonic probe, regardless of whether these ultrasonic waves are retro-reflected ultrasonic waves previously emitted by the ultrasonic probe (so called pulse-echo mode) or photoacoustically generated ultrasonic waves. The letter of these ultrasonic waves may be generated by irradiating ultrashort laser pulses or microwave pulses to the tissue of the part of the subject to be scanned. Hence, the ultrasound imaging system is presently not limited to a configuration in which ultrasound pulses are emitted and echo signals are received on the basis of an ultrasound signal obtained by means of the ultrasonic probe / ultrasound transducer.

The moving of the ultrasonic probe from the first position to the second position to at least partly scan the subject may comprise acquiring a scan, in particular, at least an A-scan, at least a B-scan and/or a C-scan when the ultrasonic probe is arranged in its second position. That is, according to this specification, the scanning may not require displacing the ultrasonic probe while the ultrasonic probe is acoustically coupled to the part of the subject. Rather, in its simplest form, scanning may be understood as acquiring an A-scan, wherein only the time-dependent pressure curve at a defined transducer/probe position and/or orientation may be acquired. The same applies for B-scans, if the ultrasonic probe comprises a linear array of ultrasonic transducers. Yet, the term scanning also encompasses a function of acquiring ultrasonic signals while displacing the ultrasonic probe transversely to an axis of the ultrasonic probe/transducer. The terms A-, B-, and C-scan are used in line with the nomenclature for sonography. That is, B-scans may be performed by scanning a single element probe along a straight line, allowing reconstructing a sectional view. Raster scanning the probe (with a single element transducer) over the sample in two orthogonal directions corresponds to a C-scan and allows for reconstructing C-scan images.

The ultrasonic probe may be configured with a single transducer (in this case preferably a point-focused transducer), a linear transducer array or a two-dimensional transducer array. In the following, the variant of the ultrasonic probe with a single transducer will be discussed, whereby this description may apply analogously for multi-transducer-element probes (i.e., probes with a plurality of transducers).

The contacting section of the patient interface device is preferably rigid. Further, the contacting section may be formed integrally with the remaining patient interface device. Accordingly, the patient interface device is preferably provided for establishing a stationary positional relationship between the part of the subject to be scanned (in the following also "the part of the subject") and a section of the ultrasound imaging system remaining stationary while the ultrasonic probe is moved for scanning the part of the subject. Thus, when the contacting section rests stationary on the subject, the contacting section may preferably be stationary in the reference system of the surrounding of the ultrasound imaging system. Alternatively, if the ultrasound imaging system is configured as a wearable device described below, the patient interface device may be stationary with the part of the subject to be scanned, but displaceable in the reference system of the surrounding. Alternative to the configuration as a wearable, the patient interface device may comprise a head rest, a chin rest and/or a forehead rest for the subject. In this case, the patient interface device may be disposed on a support, such as a table.

Preferably and regardless of whether the ultrasound imaging system is wearable, the patient interface device may comprise a housing having an opening. A main axis of the ultrasonic probe may extend through the opening. In particular, the housing may be cup-shaped or box-shaped, such that a side of the housing opposing the opening is closed. The robotic device may be (completely) arranged inside the housing. The contacting section may extend along a circumference of the opening. Thus, when the subject contacts the contacting section, the opening may be essentially closed by the subject. Thus, the subject cannot see the surrounding and is not distracted thereby. Preferably, the opening is closable by a face half of the subject. The opening may be at most 6 cm long and/or at most 6 cm wide. In an ophthalmic investigation, the subject may thus be enabled to better focus on any visual triggers (such as a light point or an image) inside the housing, in order to keep their eye to be scanned still for the ultrasonic scan. This effect may be synergistically emphasized by actively displaying an image inside the housing on a display device as described below in further detail.

In a preferred embodiment, the patient interface device is the patient-wearable device. The patient-wearable device may comprise two of the aforementioned housings so as to form goggles. In this case, the ultrasound imaging system may comprise an ultrasonic probe and a robotic device per goggle half, that is per subject eye. Synergistically, both eyes can be simultaneously imaged in this way so as to reduce the overall imaging duration. Accordingly, the control device may be configured to control both these robotic devices to move the respective ultrasonic probe from their first position to their second position to at least partly and simultaneously scan the subject. It follows that the image information conveyed to the physician can synergistically allow more profound medical investigations compared to a case in which the eyes have been imaged one after the other.

To be able to reproducibly move the ultrasonic probe under the control of the control device, the ultrasonic probe may be supported on the patient interface device only by means of the robotic device. Specifically, the ultrasonic probe may be rigidly fixed to the distal support portion of the robotic device, wherein the term distal refers to the arrangement relative to the location at which the robotic device is mounted to the patient interface device. Therefore, any movement of the distal support portion may cause an identical movement of the ultrasonic probe. Advantageously, the principles of robot control (including, in particular, respective coordinate transformations) may be applied for accurately controlling the ultrasonic probe movement, to provide precise ultrasonic data for reconstructing high resolution images.

Preferably, the robotic device is designed as a robot. Such a robot may be, in accordance with the general definition of this term, understood as a computer-programmable machine capable of carrying out a series of actions automatically. Accordingly, the control device may be part of the robotic device. Furthermore, it is preferred that the robotic device is, in sections or completely, configured as a parallel arm robot, in particular, a delta robot. The robotic device may have a length (i.e., the largest dimension) of less than 20 cm or less than 15 cm when the ultrasonic probe is in its first position. The robotic device may have a width (perpendicular to the length) of less than 20 cm or less than 15 cm when the ultrasonic probe is in its first position. The robotic device may have a width (perpendicular to the length) of less than 20 cm or less than 15 cm when the ultrasonic probe is in its first position. Thus, the overall dimensions of the robotic device may not exceed 20 cm x 20 cm x 20 cm, so that the robotic device may be compact enough to be integrated to the ophthalmic ultrasound imaging system, in particular, into the interior of the housing of the patient interface device.

The number of robotic arms may be at least 3 or at least 6. Regardless of the number of robotic arms and/or the type of robot, it is most preferred that the distal support portion (to which the ultrasonic probe may be directly attached) is mechanically connected to the base portion exclusively (i.e., only) by means of the robotic arms. When the robotic device is a parallel arm robot, all its robotic arms may be designed (essentially, i.e., within production tolerances) equally. This allows for reducing production costs and simplifying the production process. While the number of the robotic arms may be at least 3, the high speed scanning precision may be further improved by increasing the number of robotic arms. Thus, in a particularly advantageous variant, the number of the robotic arms may be at least 6. Accordingly, the robotic device may be configured as a robot with three, four, five or six degrees of freedom in which the distal support portion may be moved.

In the following, for legibility, one of the robotic arms is described in detail, wherein this description may equally apply for all the robotic arms. The robotic arm may comprise a (first) actuator, a biceps, and/or a forearm. The biceps may be arranged between the actuator and the forearm. Preferably, the forearm is connected to the actuator only by means of the biceps. The actuator (that is, the actuator of each robotic arm) may be fixedly attached to the (same) base portion. The biceps may be mounted pivotably about a respective first axis by means of the respective actuator to the base portion. In other words, the biceps may be pivotably coupled to the actuator (so as to be pivotable about (only) the first axis) and/or rotatably coupled to the forearm.

The forearm may mechanically connect the biceps with the distal support portion. Accordingly, the forearm may be rotatably coupled to the biceps and/or rotatably coupled to the distal support portion. These rotatable couplings may be configured as ball joints. Specifically, the forearm may comprise at least two linking bars (preferably extending in parallel to each other). These linking bars of the forearm may be ball-jointedly (globe-jointedly) mounted to the distal support portion and/or to the biceps. That is, the biceps may comprise a connecting section to which respective proximal ends of the linking bars may be directly coupled by means of respective first ball joints. Preferably, one ball joint is provided per linking bar. Analogously, distal ends of the linking bars may be directly coupled to the distal support portion by means of respective second ball joints. Advantageously, the degrees of freedom of the distal support portion may thus be increased.

In the above configuration of robotic arms, these robotic arms are preferably formed as parallel robotic arms so that each of these robotic arms provides a direct mechanical connection between the base portion and the distal support portion. On the other hand, in case the robot is a serial robot (serial manipulator), the distal support portion to which the ultrasonic probe may be attached, is mechanically coupled to the base portion by just one robotic arm. Thus, this robotic arm comprises a plurality of linear and/or rotational stages arranged in series to mechanically connect the distal support portion with the base portion.

It was said that the base portion is connected to the patient interface device. It is generally conceivable that the base portion is fixedly (rigidly) attached to the patient interface device, so that any displacement of the ultrasonic probe relative to the contacting section of the patient interface device is carried out by means of the robotic device. More preferably, however, the base portion may be displaceable relative to the patient interface device, in particular the housing, by means of a further actuator (second actuator). In a preferred variant, the base portion is rotatably attached to the patient interface device.

In particular, the base portion may be rotatable relative to the patient interface device about a second axis by means of a further actuator. In relation to the base portion, the second axis may extend perpendicularly to a main plane of the base portion. Also, the second axis may extend in parallel to the longitudinal axis (main axis) of the ultrasonic probe (in case of a probe having a single transducer element) or in parallel to a detection plane (in case the ultrasonic probe is configured with a linear transducer array). Alternatively or in addition to the rotatable attachment of the base portion to the patient interface device, the further actuator may be arranged for linearly displacing the robotic device along the second axis.

Advantageously, the ultrasonic probe can thus be appropriately pre-positioned for efficiently, reproducibly and accurately scanning the part of the subject, in particular, the subject's eye by means of the robotic device and the ultrasonic probe. Specifically, after fixing the patient interface device in contact with the subject, the further actuator may be controlled by the control device so as to displace the ultrasonic probe from its initial position, in which the ultrasonic probe may be retracted into the housing, to its extended position, in which the ultrasonic probe may be (by direct contact or by means of the coupling medium) acoustically coupled to the part of the subject. The initial position may be the first position of the ultrasonic probe. The extended position may be the second position of the ultrasonic probe.

To improve the scanning precision, sensor information form one or more sensors of the ultrasound imaging system may be inputted to the control device. In this regard, the ultrasound imaging system may comprise a force sensor, a proximity sensor and/or an optical sensor (e.g., a camera device, in particular, an RGB-D camera). Specifically, the distal support portion may comprise the force sensor. This sensor may be arranged to detect at least a measurement value representative of a force acting on the ultrasonic probe. In use of the ultrasound imaging system in a state arranged on the subject's face, this force may be the force between the ultrasonic probe (ultrasound probe) and the subject's eye/eyelid. The control device may receive the measurement value. The force may be an axial force. Further, the force may be determined by the control device on the basis of the measurement as a force vector. The force sensor may be configured for detecting linear and/or rotational forces acting on the ultrasonic probe in at least three, at least four, at least five or at least six directions. That is, each of these directions may be a linear direction or a rotational direction.

Further, the control device may preferably be adapted to control the robotic device to control (set) the force, by which the ultrasonic transducer is pressed against the part of the subject under investigation, so as to have a desired force value. In particular, the control device may determine whether the at least one measurement value received from the force sensor exceeds a first threshold value, and, if this is the case, displace the ultrasonic probe in a proximal direction (i.e., away from the subject / towards the base portion) until the measurement value received from the force sensor reaches a second threshold value that is smaller than the first threshold value. Vice versa, in case the measurement value drops below a third threshold value (indicative for the ultrasonic sensor lifting off of the subject), the control device may be configured to displace the ultrasonic probe in the distal direction until the third threshold value is reached. Advantageously, the distal support portion is configured as a platform. In this case, the force sensor may be integrated into the platform.

In addition or alternatively to the force sensor, a proximity sensor may be provided on the distal support portion. The proximity sensor may provide for positioning of the ultrasonic sensor at a distance from the part of the subject that is safe. Furthermore, an internal measuring unit may allow for enabling a 6D movement tracking. Moreover, if an optical sensor (in particular, the camera) is provided on the ultrasound imaging system, in particular inside the housing, optical information/image information from the optical sensor may be received by the control device. A depth sensor may be comprised in the ultrasonic imaging system. In particular, the depth sensor may be part of the optical sensor. Preferably, the optical sensor may be an RGB-D sensor. Thus, the optical sensor may acquire under the control of the control device not only (optical) image information, but also depth information. Accordingly, the control device and robotic device may be configured to cooperate so as to enable safely approximating the subject and detecting a head anatomical structure of the subject.

To further increase safety for the subject, an inertial measurement device, in particular, configured as an inertial measurement unit (IMU) may be provided on the ultrasound imaging system, particularly on the patient interface device. Information from the each of these sensor devices (i.e., the force sensor, the proximity sensor, the optical sensor (camera) and/or the inertial measurement device) may be combined by the control device by means of sensor-data-fusion. The control device may be adapted for following a movement of the subject by means of sensor-data-fusion. Thus, safety may be further increased.

Using the optical sensor, in particular the RGB-D camera, calibrated with the robotic device's end-effector, a 3D model of the subject's (patient's) face may be created. On the basis of data representing this 3D model, patient-dependent data of anatomical features like nose size, eyebrow shape, and eye position may be precisely measured. Further, based on this patient-dependent data, the ultrasound imaging system may (adaptively) adjust the scanning area, prevent collisions in real-time, and refine robotic control to ensure high precision.

Real-time proximity sensing may provide continuous feedback on a distance between the ultrasonic probe and the facial anatomy of the subject. Thus, the following advantages may be achieved:
- Monitoring positioning: Continuously tracking the position of the ultrasonic probe relative to the face may ensure accurate control over the scanning process.
- Detecting collisions: Collisions or interactions between the ultrasonic probe and (especially delicate) facial structures may be proactively identified.
- Adapting dynamically: With real-time proximity sensing, the ultrasound imaging system may adaptively adjust its behavior in response to changing circumstances. Thus, risks may be minimized, and safe and precise scanning procedures may be ensured.

Moreover, the ultrasound imaging system may comprise the aforementioned display device. The display device may be arranged in the vicinity of the ultrasonic probe. Further, the display device may be visible through the opening of the housing. The control device may be configured to control the display device to display an image that may be visible through the opening. The image may comprise, for example, a symbol, a letter, and/or a pattern. In particular, the display device may comprise a display surface on which an image may be displayed. The display surface may preferably be arranged such as to be visible for a subject/patient with a line of sight parallel to the longitudinal axis of the ultrasonic probe and looking towards the ultrasonic probe. Furthermore, an audio feedback for the subject may be provided.

Thus, during the scanning controlled by the control device, the control device may be configured for attracting the subject's line of sight to the displayed image, i.e. causing the subject to look in a desired direction. Similarly, the display may be used for causing the eye to hold still during the scan controlled by the control device. Eyeball-navigation for ocular ultrasound can be improved by the display. Synergistically, the housing may be opaque (impervious to light), so that when the opening in the housing is closed by the subject during the scan, the image may be the major/most prominent visual trigger for the patient. Thus, patient comfort and safety may be increased.

In addition, the ultrasound imaging system may comprise a battery system providing autarky. Also, the ultrasound imaging system may have a communication device configured for bidirectional communication with a remote control system (e.g., a computer device). For example, an integrated internet-of-things (IOT) configuration may enable bi-directional communication, so that the ultrasound data may be sent to the remote control system. A physician may remotely control the ultrasound imaging system / robotic device by means of the remote control system communicating with the control device of the ultrasound imaging system. Thus, the ultrasound imaging system according to the present invention is suitable for telemedicine.

For imaging the part of the subject by means of the ultrasound imaging system, the subject may be brought into contact with the contacting section of the patient interface device. While this contact is maintained (for example by fixing the patient interface device to the patient), the control device may control with the robotic device, in particular, the first actuators and/or the second actuator, to displace the ultrasonic probe from the first position, preferably axially, to the second position. In the first position, the ultrasonic probe may be retracted into the housing of the patient interface device and/or uncoupled ultrasonically from the part of the subject.

At least when the ultrasonic probe is located in the second position, the control device may control the detection of ultrasonic waves incident on the ultrasonic probe by means of the ultrasonic probe to generate an ultrasonic signal, and may process this ultrasonic signal to generate at least an A-scan of the part of the subject. The control device may further be adapted to control scanning the ultrasonic probe relative to the patient interface device according to a pattern to generate a sonographic image data set comprising image data of at least an A-scan, at least a B-scan and/or at least a C-scan. Specifically, the control device may be configured to control the robotic device to displace the ultrasonic probe (preferably according to the pattern) along the contour of the part of the subject (eye), such as along the surface of the cornea, while acquiring ultrasonic signals, to generate the sonographic image data set. In a preferred variant, the at least partly scanning the object may comprise identifying such an eye landmark in a first scan at the second position (e.g., the A-scan or the B-scan (image)), most preferably by segmentation, and further displacing the ultrasonic probe on an imaginary sphere with center on an eye landmark while acquiring ultrasonic signals of the sonographic image data set. The eye landmark may be, for example, an eye landmark on the eye fundus, most preferably the fovea.

Further, the control device may be configured to reconstruct one or more B-scan images, one or more C-scan images, and/or one or more three-dimensional (3D) images on the basis of the sonographic image data set. In other words, the control device may thus be adapted to generate, preferably in real-time, a three-dimensional ultrasound image on the basis of the sonographic image data set. For reconstructing the A-, B- and/or C-scan images, for example, a back-projection algorithm may be utilized. The reconstructed A-, B- and/or C-scan images may be output for display on a further display outside the housing of the patient interface device, so as to be viewed by the physician. Preferably, these images may be reconstructed and displayed in real-time, that is, while the respective scan is being performed. In a particularly advantageous embodiment, the control device may be configured for reconstructing the three-dimensional image of the eye of the subject on the basis of a plurality of B-scan images that may preferably be registered on the basis of the eye landmark.

Further advantageous features of the ultrasound imaging system, in particular, the group of sensor devices, may comprise:
1. The ultrasound imaging system may comprise a camera sensor: Real-time RGB/D image extraction with the calibrated image sensor on the robot's end-effector may help to provide a safe and robust approach to the patient's eye, utilizing the already analyzed anatomical features of the patient's face.
2. The inertial measurement unit may be configured as a 9-DOF (9 degrees of freedom) IMU sensor integrated into the robotic device to provide comprehensive motion and geometrical information about the head and body of the subject.
3. The proximity sensor may be configured as an infrared (IR) or time-of-flight (ToF) sensor. This sensor allows for ensuring that the distance between the robot and its end-effector is safe, enabling non-collision approaches.
4. Visual and audio feedback may be provided. The visual display may provide visual instructions for the subject. An integrated audio system may notify the patient of any required instructions while scanning, for example, to rotate the eyeball and look to the left to be able to extend the scanable field of interest, e.g., to the peripheral area of the eyeball.

Returning to the variant in which the robotic device is the serial robot with just one robotic arm, the robotic arm may comprise a plurality of linear stages and/or rotational stages connected in series. These linear and/or rotational stages may be formed as mini-stages (with the overall dimensions of the robotic device being at most 8 cm by at most 8 cm by at most 8 cm and/or the maximum linear travel of each linear stage being for example at most 5 cm or at most 4 cm) which may comprise actuators (for example, stepper motors).

A (most-proximal) linear stage may be fixed to the patient interface device to provide a stationary relationship between the contacting surface of the contacting section and the ultrasonic probe. Further, in this variant, the ultrasonic probe preferably comprises a linear array of ultrasonic transducers, so as to be able to essentially simultaneously acquire image data of several A-scans (i.e., a B-scan). Furthermore, the proximity sensor may be, in an axial view (relative to the axial direction of the ultrasonic probe) ring-shaped, and the ultrasonic probe may extend through this ring.

The method of imaging suggested herein may be a method of imaging at least part of a subject by an ultrasound imaging system, preferably the ultrasound imaging system described in detail above. The method comprises the step of moving the ultrasonic probe from the first position to the second position, and scanning the subject at least partly (i.e., scanning at least a part of the subject). The scanning of the subject may be performed while the ultrasonic probe is arranged in the first position and/or the second position.

Preferably, the part of the subject may be an eye of the subject. Accordingly, the method of imaging may be an ophthalmic ultrasound imaging method. The method may be carried out intraoperatively. Thus, a medical or surgical instrument may be attached to the distal support portion. The control device may thus be configured to manipulate the instrument.

Furthermore, the method may comprise as method steps any, in particular each, of the functional features described above in detail in the context of the ultrasound imaging system. Moreover, the method may comprise any, in particular each, of the features of the ultrasound imaging system described above in detail.

For example, the robotic device may be controlled by the control device to set the force to a desired force value. Moreover, the display device may be controlled by the control device to display the image. The ultrasonic probe may be scanned relative to the patient interface device according to the pattern to generate the sonographic image data set comprising data of at least the A-scan, at least the B-scan and/or at least the C-scan. A three-dimensional ultrasound image may be generated, preferably in real-time, on the basis of the sonographic image data set.

Preferred embodiments of an ultrasound imaging system and a method of imaging will now be explained in greater detail with reference to the attached schematic drawings, wherein
- Figure 1: shows a first variant of an ultrasound imaging system in a side view, wherein the housing is omitted and the ultrasound probe is located in its second position;
- Figure 2: shows the ultrasound imaging system of figure 1 and its spatial relationship to the part of the subject to be scanned in a perspective view;
- Figure 3: shows the ultrasound imaging system of figure 1 with a viewing direction through the opening of the housing;
- Figure 4: shows the ultrasound imaging system of figure 1 in an axial view;
- Figure 5: shows the ultrasound imaging system of figure 1 in a lateral view;
- Figure 6: shows the ultrasound imaging system of figure 1 in a further view;
- Figure 7: shows the robotic arm of the ultrasound imaging system of figure 1 in a detailed perspective view;
- Figure 8: shows the ultrasound imaging system of figure 1 as fixed to the subject;
- Figure 9: shows a second variant of an ultrasound imaging system in a perspective view, wherein the housing is omitted and the ultrasonic probe is located in its second position;
- Figure 10: shows the ultrasound imaging system of figure 9 in a side view;
- Figure 11: shows the ultrasound imaging system of figure 9 during the scanning of the subject's eye;
- Figures 12 and 13: show block diagrams illustrating functionalities of the ultrasound imaging system of figure 1; and

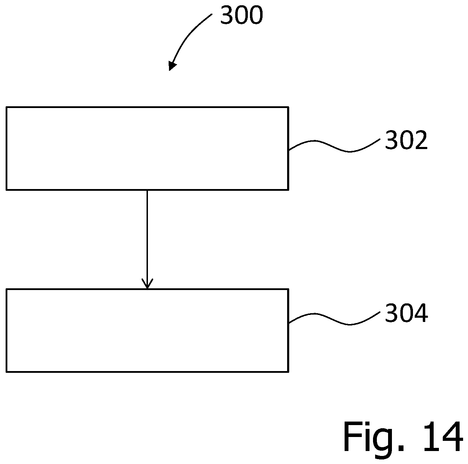

- Figure 14: shows a method of imaging the part (eye) of the subject by means of the ultrasound imaging system of figure 1.

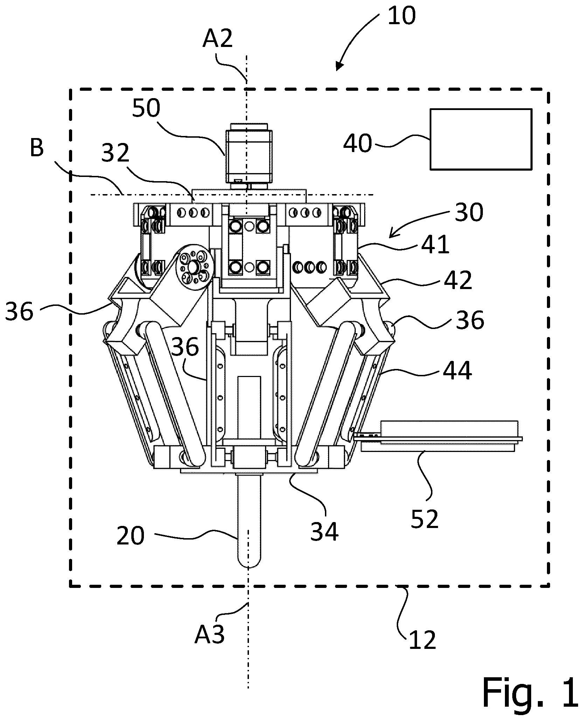

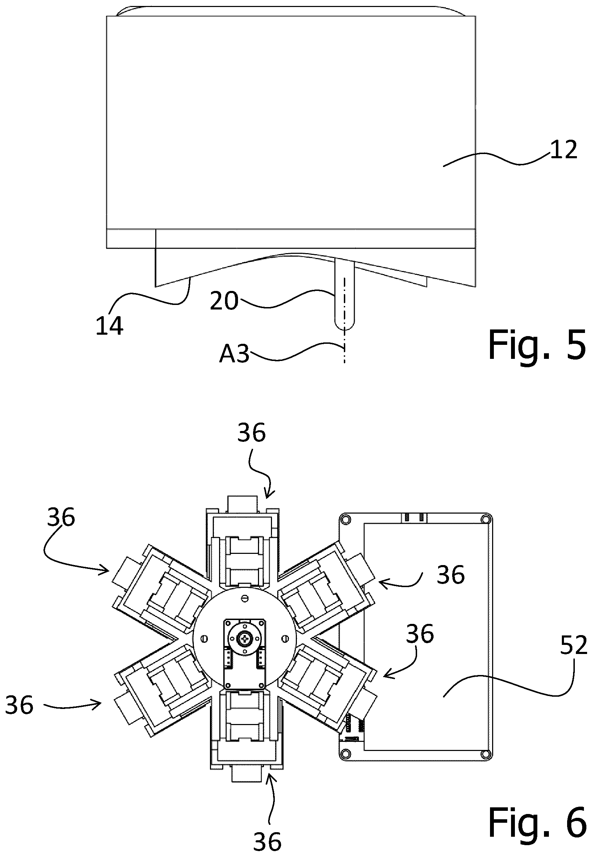

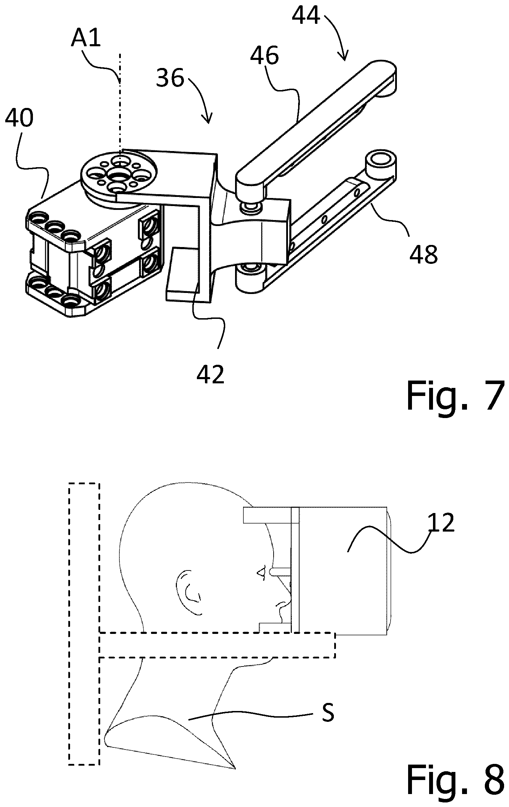

Figures 1 to 8 show an ultrasound imaging system 10. The ultrasound imaging system 10 is preferably configured for scanning an eye of a patient (subject S). I.e., the ultrasound imaging system 10 may be an ophthalmic ultrasound imaging system. The ultrasound imaging system 10 comprises a patient interface device 12, an ultrasonic probe 20, a robotic device 30, as well as a control device 40.

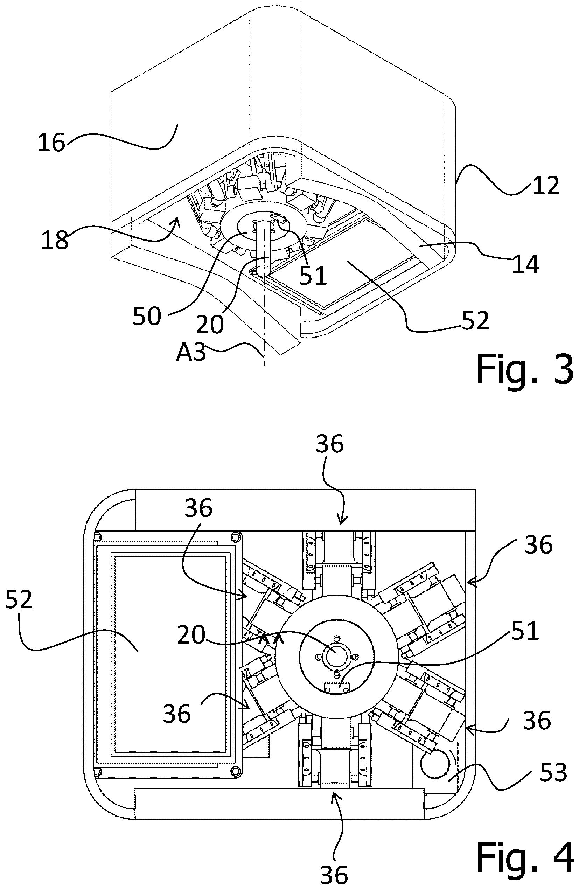

The patient interface device 12 comprises a contacting section 14 for contacting the subject S (see figure 3). As shown in figures 3 to 5 in further detail, the patient interface device 12 is shaped as a cup, that is, a housing 16 having an opening 18 on one side. In the views of figures 1 and 3, this side is the bottom side / distal side of the ultrasound imaging system 10. The distal side is generally the side facing the subject S when the patient interface device 12 is fixed to the subject S during the intended use. The term proximal side refers to the side of the patient interface device 12 opposing the distal side. The patient interface device 12 is a wearable device, but may alternatively be a stationary device (e.g., disposed on a table). In the latter case, the patient interface device 12 may comprise a head rest, a chin rest and/or a forehead rest for the subject S.

The ultrasonic probe 20 is presently a so-called single element probe, i.e., the ultrasonic probe 20 comprises a single transducer, which may preferably be point focused or unfocused. The control device 40 may be configured for filtering the ultrasonic signals acquired by the transducer. The transducer may be a piezoelectric transducer. In modifications, the ultrasonic probe 20 may comprise more than one transducer; these transducers may preferably be arranged in a one-dimensional or two-dimensional transducer array. Regardless of the number of transducer elements of the ultrasonic probe 20, the main (emission and/or detection) axis A3 of each transducer element may be oriented transversely, in particular perpendicularly, to the opening of the housing, so that the ultrasonic probe 20 may scan the object through the opening 18. That is, as shown in figure 3, the main axis A3 may extend through the opening 18. Preferably, the ultrasonic probe 20 may be arranged to extend through the opening 18. I.e., the ultrasonic probe 20 may project to the outside of the housing 16 through an imaginary plane defined by the surface of the contacting section 14 (on which the subject S contacts the patient interface device 12 during intended use). Thus, the contacting section 14 may be curved to better match the contour of the subject's face.

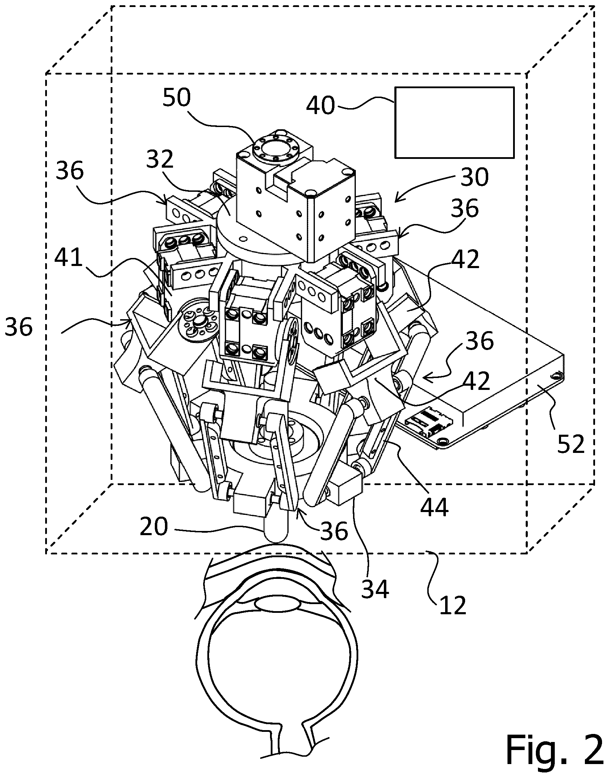

This robotic device 30 is configured as a parallel arm robot (also called parallel robot), and comprises a base portion 32 and a distal support portion 34 spaced from the base portion 32. The ultrasonic probe 20 is fixedly connected to the distal support portion 34. The distal support portion 34 (so called end-effector of the robot) is movably coupled to the base portion 32 by means of (preferably at least three to six) robotic arms 36 formed/designed equally. The distal support portion 34 is herein configured as a platform. When the robotic device 30 is a delta robot, the number of robotic arms may be 3. The base portion 32 is connected to the patient interface device 12. The control device 40, is configured to control the robotic device 30 to move the ultrasonic probe 20 from a first position to a second position shown in figure 2 to at least partly scan the subject S (at least in the second position).

The distal support portion 34 is mechanically connected to the base portion 32 exclusively by means of the robotic arms 36. Each of the robotic arms 36 comprises an actuator 41, a biceps 42 and a forearm 44. The respective biceps 42 is mounted pivotably about a respective first axis A1 by means of the respective actuator 41 to the base portion 32. Linking bars 46, 48 of the forearm 44 are ball-jointedly mounted to the distal support portion 34 and/or to the biceps 42. The base portion 32 is rotatable relative to the patient interface device 12 about a second axis A2 extending perpendicular to a main plane B of the base portion 32 by means of a further actuator 50. Thus, the ultrasonic probe 20 is movable with six degrees of freedom.

The ultrasound imaging system 10 further comprises a display device 52 arranged in the vicinity of the ultrasonic probe 20 and the control device 40 is configured to control the display device 52 to display an image. The image may be a pattern or the like, and may draw the subject's attention so as to define the line of sight. The distal support portion 34 comprises a force sensor 50 arranged to detect at least a measurement value representative of a force acting on the ultrasonic probe 20. The control device 40 is preferably adapted to control the robotic device 30 to set the force to a desired force value. The force sensor 50 is integrated into the platform. The force sensor 50 may be a 6 degrees-of-freedom (6-DOF) force sensor.

Moreover, the ultrasound imaging system 10 may comprise a proximity sensor 51 for facilitating safely setting the distance between the ultrasonic probe 20 and the subject's head. An optical sensor 53 may be formed as a camera and may, under the control of the control device 40, enable safe approximation to the patient's eye and detection of the patient's head anatomical structure. The distal support portion may preferably further comprise an inertial measurement unit (not shown) that may be integrated into the distal support portion 34 (that is the end-effector). The force sensor 50, the proximity sensor 51 and/or the optical sensor 53 may form a sensing section of the ultrasound imaging system 10. The sensor devices of this sensing section may provide the control device with information such that the control device 40 may control following a movement of the subject S by means of sensor-data-fusion.

The control device 40 is adapted to control scanning the ultrasonic probe 20 relative to the patient interface device 12 according to a pattern to generate a sonographic image data set comprising (image data of) at least an A-scan, at least a B-scan and/or at least a C-scan. Specifically, the control device 40 may be configured to displace the ultrasonic probe 20, in particular axially, from its first position to its second position, and acquire an A-scan of the subject's eye at least when the ultrasonic probe 20 is located in its second position (see figure 2). Furthermore, the control device 40 may control the robotic device 30 to further displace the ultrasonic probe 20 transversely (relative to the axial direction) in a first direction, for example parallel to the cornea, and optionally in a second direction that is non-axial and at an angle to the first direction and the second direction to acquire the sonographic image data set so as to comprise a plurality of A-scans or, respectively, a plurality of B-scans. Thus, the control device 40 may be adapted to generate, preferably in real-time, a three-dimensional ultrasound image on the basis of the sonographic image data set. The ultrasound imaging system 10 may further comprises a communication device configured for bidirectional communication to send the sonographic image data set or any of the reconstructed images, such as the three-dimensional image, to a remote computer device for display.

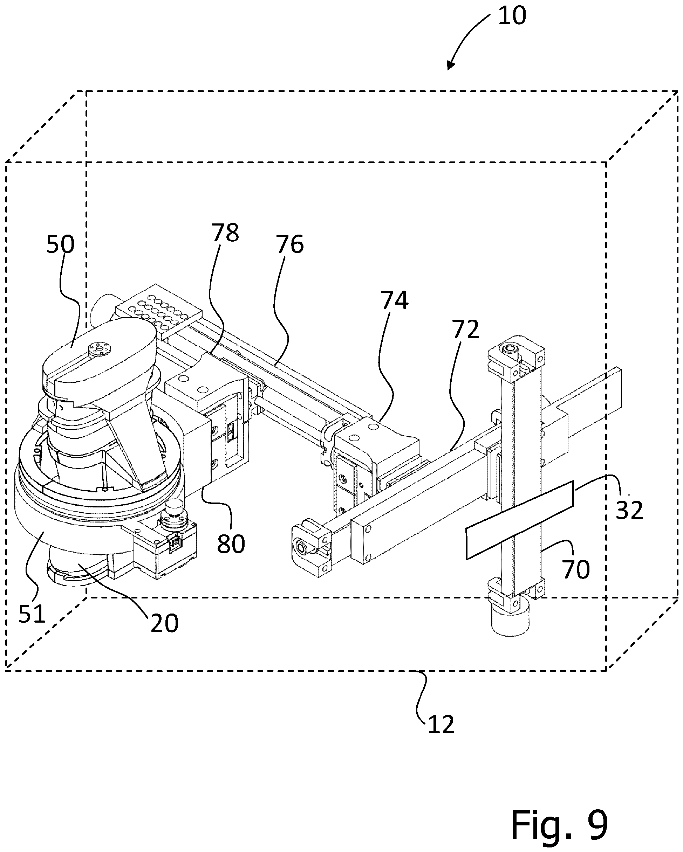

An ultrasound imaging system 10 according to figure 9 differentiates from the ultrasound imaging system 10 of figure 1 by the robotic device 30 being a serial robot with just one robotic arm 36. The robotic arm 36 comprises a plurality of linear stages 70, 72, 76 and/or rotational stages 74, 78, 80 connected in series. These linear and/or rotational stages may be formed as mini-stages (with the overall dimensions of the robotic device being at most 8 cm by at most 8 cm by at most 8 cm and/or the maximum linear travel of each linear stage being for example at most 5 cm or at most 4 cm) comprising respective actuators (for example, a stepper motors).

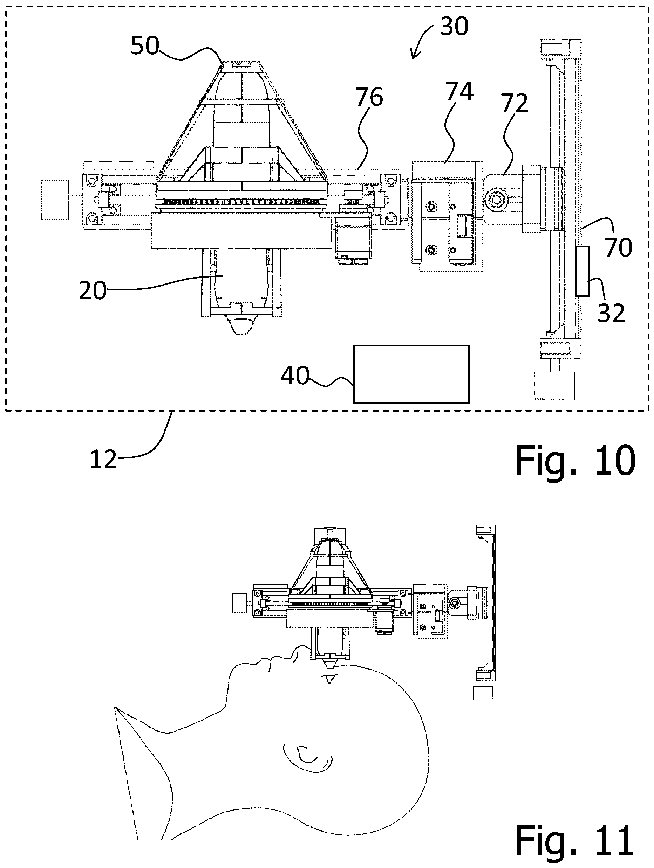

The linear stage 70 is fixedly attached to the patient interface device to provide a stationary relationship between the contacting surface of the contacting section 14 and the ultrasonic probe 20. Further, in this variant, the ultrasonic probe 20 comprises a linear array of ultrasonic transducers, so as to be able to essentially simultaneously acquire several A-scans (i.e., a B-scan). Furthermore, the proximity sensor 51 may be, in an axial view (relative to the axial direction of the ultrasonic probe 20) ring-shaped, and the ultrasonic probe may extend through this ring. Like in the variant of figure 1, the proximity sensor 51 may be a 6D proximity sensor. Thus, "off the shelf" comparably large ultrasonic probes that physicians are familiar with, may be accommodated by the robotic device 30. A situation in which the ultrasonic probe 20 is located in its second position is shown in figure 11. Furthermore, the ultrasound imaging system 10 of figure 9 may comprise all the features of the ultrasound imaging system 10 of figure 1.

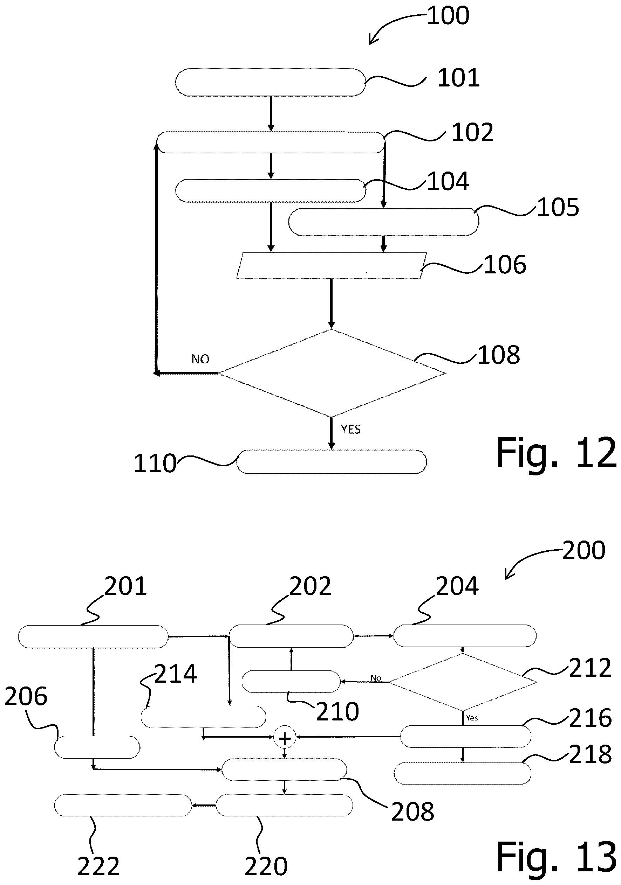

Figures 12 and 13 show block diagrams representing possible functionality of the ultrasound imaging system 10 of figure 1, in particular, method steps controlled by the control device 40. Specifically, an initialization functionality/method (robot-eye initialization) that the control device 40 is adapted to control is shown in figure 12. After the start 101 of this method, a robot initialization three-dimensional movement is carried out at 102 by means of the actuators 41, 50 controlled by the control device 40. Camera frames of the camera are extracted at 104. Preferably in parallel thereto, at 105, the force sensor 50 is being read-out. The camera frames and/or information representative for the camera frames are processed in a data fusion layer at 106. The control device 40 then determines, on the basis of this information, at 108 whether placement of the ultrasonic probe 30 in the second position is safe. If the control device determines that this is the case ("YES"), the initialization functionality/method is completed (at 110). Otherwise, steps 102 to 108 are repeated until the result at 110 is positive ("YES").

The scanning functionality/method ("robot-eye scanning") that the control device 40 is adapted to control is shown in figure 13. At 200, an eyeball navigator process may start with utilizing the optical sensor 53 (in particular, camera) to detect the part of the subject, in particular the eye, segment the eyelid or eye and reconstruct the anatomical features of the patient's face. At 206, eyelid/eye position in the camera frame with respect to the robotic device's end-effector (distal support portion 34) may define initiation of the robotic device in an appropriate distance. At 202, by utilizing a collision avoidance process, the robotic device may start scanning the eye. At 204, a real-time tissue awareness process which may use real-time F/T integrated force sensor information may maintain an appropriate force on the eye (212). If the applied force is not high enough or exceeds a predefined (safe) value, the compensation process 210 may start to fine-tune the position of the ultrasonic probe 20. However, at 216, if the applied force as detected is in a (desired) predetermined range, the related ultrasound B-scan may be extracted. At the same time, the 6D pose of the robotic device may be extracted (214).

At 218, a real-time AI-based segmentation and classification network may be utilized to on-line segment the eyeball layers and optic nerve. Thus, high quality of the extracted B-scan may be achieved. Further, the real-time AI-based segmentation and classification network may start annotating a diagnosis label to be later used as an attention layer for a data-fusion layer at 208. At 208, the data fusion layer, may fuse the extracted B-scan data, robotic device's 6D pose, AI-based nerve and layers segmentation and disease detection, to reconstruct an accurate 3D-scan of the eyeball at 220. At eyeball rendering process 222, the reconstructed 3D-scan may be computed with more additional post-processing and be visualized on the display device 52, preferably in a graphical user interface (GUI), or be sent to an ultrasound expert located remotely.

In figure 16, a method 300 of imaging at least part of a subject S by an ultrasound imaging system 10 is shown schematically. This method comprises a step 302 of moving the ultrasonic probe 20 from the first position to the second position. In a further step 304, the subject S can be scanned at least partly. The step 302 may comprise any of the features of method 100. The step 304 may comprise any of the steps of method 200.

The terms "comprising", "having", "with" and the like used in this disclosure are to be understood as non-limiting. In particular, the term "comprising a" means in this context "comprising at least a", i.e., "comprising a" does not exclude the presence of further corresponding elements. In the present case, at least a/one means one or more. For reasons of readability, the expression "at least" is partly omitted in this disclosure to simplify. If a feature of the present disclosure is described in the singular or indefinite, the plurality thereof should also be disclosed at the same time. At least in sections/in parts, it is to be understood as in sections/in parts or completely.

## Claims

1. An ultrasound imaging system (10), comprising
a patient interface device (12) having a contacting section (14) for contacting a subject (S),
at least an ultrasonic probe (20),
at least a robotic device (30) having a base portion (32) and a distal support portion (34) spaced from the base portion (32), and
a control device (40),
wherein the ultrasonic probe (20) is fixedly connected to the distal support portion (34),
wherein the distal support portion (34) is movably coupled to the base portion (32) by means of one or more robotic arms (36),
wherein the base portion (32) is connected to the patient interface device (12), and
wherein the control device (40) is configured to control the robotic device (30) to move the ultrasonic probe (20) from a first position to a second position to at least partly scan the subject (S).

2. The ultrasound imaging system (10) according to claim 1,
wherein the robotic device (30) is designed as a robot, in particular, a parallel arm robot, a delta robot or a serial robot.

3. The ultrasound imaging system (10) according to claim 1 or 2,
wherein all robotic arms (36) are designed equally,
and/or wherein a number of the robotic arms (36) is at least 3 or at least 6.

4. The ultrasound imaging system (10) according to any one of the preceding claims, wherein the distal support portion (34) is mechanically connected to the base portion (32) exclusively by means of the robotic arms (36).

5. The ultrasound imaging system (10) according to any one of the preceding claims, wherein the robotic arms (36) each comprise an actuator (41), a biceps (42) and a forearm (44).

6. The ultrasound imaging system (10) according to the preceding claim,
wherein the respective biceps (42) is mounted pivotably about a respective first axis (A1) by means of the respective actuator (41) to the base portion (32),
and/or wherein linking bars (46, 48) of the forearm (44) are ball-jointedly mounted to the distal support portion (34) and/or to the biceps.

7. The ultrasound imaging system (10) according to any one of the preceding claims, wherein the base portion (32) is rotatable relative to the patient interface device (12) about a second axis (A2) extending perpendicular to a main plane (B) of the base portion (32) by means of a further actuator (50).

8. The ultrasound imaging system (10) according to any one of the preceding claims,
wherein the distal support portion (34) comprises a force sensor (50) arranged to detect at least a measurement value representative of a force acting on the ultrasonic probe (20),
wherein the control device (40) is preferably adapted to control the robotic device (30) to set the force to a desired force value.

9. The ultrasound imaging system (10) according to any one of the preceding claims, wherein the distal support portion (34) is configured as a platform and the force sensor (50) is integrated into the platform.

10. The ultrasound imaging system (10) according to any one of the preceding claims,
wherein the patient interface device (12) comprises a housing (16) with an opening (18), and
wherein a main axis (A3) of the ultrasonic probe (20) extends through the opening.

11. The ultrasound imaging system (10) according to any one of the preceding claims,
wherein the patient interface device (12) is a wearable device,
and/or wherein the ultrasound imaging system is an ophthalmic ultrasound imaging system,
and/or wherein the ultrasound imaging system (10) further comprises a display device (52) arranged in the vicinity of the ultrasonic probe (20) and the control device (40) is configured to control the display device (52) to display an image.

12. The ultrasound imaging system (10) according to any one of the preceding claims,
wherein the patient interface device (12) comprises a head rest, a chin rest and/or a forehead rest for the subject (S).

13. The ultrasound imaging system (10) according to any one of the preceding claims,
wherein the control device (40) is adapted to control scanning the ultrasonic probe (20) relative to the patient interface device (12) according to a pattern to generate a sonographic image data set comprising at least an A-scan, at least a B-scan and/or at least a C-scan.

14. The ultrasound imaging system (10) according to any one of the preceding claims,
wherein the control device (40) is adapted to generate, preferably in real-time, a three-dimensional ultrasound image on the basis of the sonographic image data set.

15. The ultrasound imaging system (10) according to in assumed inclining any one of the preceding claims, further comprising:
a communication device configured for bidirectional communication,
and/or one or more sensor devices, wherein the control device is adapted for following a movement of the subject by means of sensor-data-fusion.

16. A method (300) of imaging at least part of a subject (S) by an ultrasound imaging system (10) according to one of the preceding claims, comprising the step of moving (302) the ultrasonic probe (20) from the first position to the second position, and scanning the subject (S) at least partly.
